# EUROPEAN PATENT SPECIFICATION

(11) **EP 1 255 456 B1**
(45) Date of publication and mention of the grant of the patent: **12.12.2007**
(21) Application number: 01908450.8
(22) Date of filing: 08.02.2001
(51) Int. Cl.: A23L 1/305, A61K 31/195

(54) **USE OF GLUTAMATE OR OF GLUTAMIC ACID FOR THE PREVENTION OF HYPERPERMEABILITY OR UNDESIRED PERMEABILITY OF THE INTESTINAL WALL**
VERWENDUNG VON GLUTAMAT ODER GLUTAMINSÄURE FÜR PRÄVENTION VON HYPERPERMEABILITÄT ODER UNGEWÜNSCHTER PERMEABILITÄT DER DARMWAND
UTILISATION DE GLUTAMATE OU D'ACID GLUTAMIQUE POUR PREVENIR L'HYPERPERMEABILITE OU LA PERMEABILITE NON-SOUHAITEE DE LA PAROI INTESTINALE

(30) Priority: 14.02.2000 NL 1014380
(43) Date of publication of application: 13.11.2002
(73) Proprietor: Friesland Brands B.V., 8901 MA Leeuwarden (NL)
(72) Inventor: VAN LEEUWEN, Paulus, Aluisius, Marie, NL-1187 VN Amstelveen (NL); HOUDIJK, Alexander, Petrus, Johannes, NL-1181 RS Amstelveen (NL); GLAS, Cornelis, NL-9255 KE Tietjerk (NL)
(74) Representative: van Westenbrugge, Andries
(86) International application number: PCT/NL2001/000104
(87) International publication number: WO 2001/058283

(56) References cited:
- US-A- 5 366 723
- US-A- 5 981 590
- RAUL FRANCIS ET AL: "Functional and metabolic changes in intestinal mucosa of rats after enteral administration of ornithine alpha-ketoglutarate salt." JOURNAL OF PARENTERAL AND ENTERAL NUTRITION, vol. 19, no. 2, 1995, pages 145-150, XP001001310 ISSN: 0148-6071
- EWTUSHIK A L ET AL: "Performance and intestinal development of piglets receiving diets supplemented with selected amino acids or polyamines." CANADIAN JOURNAL OF ANIMAL SCIENCE, vol. 77, no. 4, December 1997 (1997-12), pages 741-742, XP001001415 1997 Annual Meeting of the Canadian Society of Animal Science ISSN: 0008-3984
- DATABASE WPI Section Ch, Week 199342 Derwent Publications Ltd., London, GB; Class B04, AN 1993-330544 XP002148350 & JP 05 236909 A (SNOW BRAND MILK PROD CO) cited in the application
- PATENT ABSTRACTS OF JAPAN vol. 1996, no. 10 & JP 08 157385 A (KIRIN BREWERY) cited in the application
- EDITOR: KATHLEEN PARFITT: "Martindale 32" 1999, PHARMACEUTICAL PRESS , LONDON, UK * page 1344 - page 1345 *

## Description

The invention relates to the preparation of a nutritional preparation that is suitable for use in the case of conditions associated with an increased permeability of the intestinal wall.

The intestinal epithelium acts as a selective barrier which allows the absorption of nutrients but restricts the passage of microorganisms and undesired macromolecules. Maintaining this barrier is considered to be important in order to protect the host against the migration of pathogenic microorganisms from the intestines to the bloodstream. It is assumed that the increase in the permeability of the intestines is associated with damage to the paracellular transport system of the intestinal mucosa, as a result of which translocation of endotoxins and (pathogenic) bacteria can occur. As a result of the damage to the intestinal mucosa it is also possible for absorption of macromolecules to occur, which are then able to initiate allergic reactions.

An increase in the permeability of the intestinal wall has been detected in clinical conditions associated with damage to the intestinal mucosa barrier, such as endotoxaemia, sepsis, multiple trauma, malnutrition, major surgical interventions, parenteral nutrition and bums. An increase in the permeability to larger molecules, such as proteins, has been found in the newborn, but also occurs in healthy people if they are allergic to food products.

It is known that glutamine is able to lower the macromolecular hyperpermeability of intestinal cells which is induced by phorbol 12,13-dibutyrate (Kouznetsova et al. J. Parenteral Enteral Nutrition, 23 (1999) 136-139). A disadvantage of glutamine, however, is that it is not stable at room temperature, which renders it unsuitable for (non-chilled) foods with a long shelf life. Moreover, glutamine has poor solubility.

Instead of glutamine, specific products based on peptides, mainly di- and tripeptides, are used. These peptides are frequently prepared from glutamine-rich vegetable proteins, such as, in particular, wheat protein, in which preparation method, following enzymatic conversion, fractionation technology is used in order to obtain the specific peptide fraction as the main fraction. Examples thereof can be found in JP 05236909 and JP 08157385. Such a preparation of these peptides is expensive and complex. Moreover, a product obtained from wheat protein can be problematic for some patients, such as coeliac patients.
Raul et al. (J of Parenteral and Enteral Nutrition, 1995, Vol. 19: 145-150) describe that the combination of ornithine alpha-ketoglutarate promotes growth and differentiation of intestinal mucosal cells.
Ewtushik et al. (Canadian J of Animal Science 1997, Vol. 77: 741-742) describe the effect of feeding weaned piglets with food compositions supplemented with various amino acids and the effects on piglet growth.
JP 05 236909 (Snow Brand Milk Production) discloses the use of compositions containing glutamic acid as a high calorie transfusion for weight gain of patients.
JP 08 157385 (Kirin Brewery) describe compositions containing glutamic acid and glutamine for enhancing mucosal cell growth.
US 5,366,723 describe compositions for the regeneration of the mucosa, wherein glutamic acid, aspartic acid and cysteine are used.
Both US 5,981,590 and J. Parenteral Nutrition (1999, No. 23: 136-139) studied the effects of glutamine on intestinal hyperpermeability.
The handbook Martindale 32, 1999, Kathleen Parfitt; editor; Pharmaceutical Press London, UK) describe the use of glutamine in patients with injuries of burns or infections.

It has now been found that too high a permeability of the intestinal wall can be effectively treated or prevented by the administration of a suitable quantity of glutamate and/or glutamic acid, preferably in a nutritional preparation. The invention is defined in claim 1 and in the thereof depending claims.

From the state of the art, for instance US 5,366,723 it is known to use a combination of glutamic acid, aspartic acid and cystein in decreasing the toxicity of platinum compounds in the treatment of cancer. One of the activities mentioned is regeneration of the intestinal mucosa. However, regeneration of the intestinal mucosa relates to a different phenomena than permeability of the intestine which is in particular associated with integrity of the intestine or the paracellular transport via the intestinal mucosa.

Here a nutritional preparation is understood to be a composition that contains food constituents (at least one), such as proteins, carbohydrates, fats, vitamins, minerals and the like. Preferably the composition contains more than one food constituent and preferably it contains all necessary food constituents. It can therefore be a food supplement or a complete food or a food drug ("neutraceuticum").

The nutritional preparation of the invention can be an infant formula or children's food or an enteral (functional/clinical/problem solving) food.

Substances that are known to have a beneficial effect on the intestinal function (permeability) can also be added. In particular, one or more polyamines such as spermine, spermadine or putrescine or one or more polyamine precursors, in particular omithin and arginin can be used. Such polyamines are, for example, described in Dorhout et al., Br. J. Nutrition, 1997, 639-654 and in a report of a seminar held on 29 June to 2 July 1999 in Glasgow, published in Proceedings of the Nutrition Society, Vol. 59 (Issue 1), 2000, 81-86. Polyamines or the precursors thereof can have a beneficial contribution in e.g. postnatal intestinal maturation, permeability of the intestine to macromolecules (allergy), the translocation of bacteria, etc.

The preparation contains preferably 0.05 to 10 g, more preferably 0.2 to 4 g free glutamate per 100 g of the nutritional preparation (dry weight). The polyamines are preferably present in an amount of 1 to 10 mg per 100 g of the nutritional preparation (dry weight).

The glutamate is preferably incorporated in a nutritional preparation alongside proteins or peptides, such as lactic or vegetable proteins. The nutritional preparation contains in particular lactic proteins or hydrolysates obtained therefrom. Lactic proteins comprise casein, whey proteins and lactoferrin.

Carbohydrates are understood to be digestible carbohydrates, such as glucose, lactose, maltose and sucrose, and digestible oligosaccharides and polysaccharides, such as maltodextrins, amylopectins and starch, as well as non-digestible carbohydrates (food fibres) such as galacto-oligosaccharides or fructo-oligosaccharides (inulin), vegetable and animal and microbial gums, such as carob bean flour and gum arabic. Fats comprise vegetable and animal fats, fats with medium length chains (C₈-C₁₂) (MCT), fats with unsaturated long chains (such as γ-linolenic acid, arachidonic acid, eicosapentaenoic acid and docosahexaenic acid).

The nutritional preparation according to the invention can also contain glutamine or an equivalent thereof. Glutamine equivalents are known to those skilled in the art. Examples of these are the abovementioned dipeptides and tripeptides. If the nutritional preparation is a food for babies or toddlers, the weight ratio of glutamic acid : glutamine from the free amino acids is greater than 1 : 1, in particular greater than 5 : 1 and very particularly greater than 25 : 1.

Glutamate can be in the form of a physiologically acceptable glutamate salt (for example the sodium, potassium, calcium or magnesium salt). As a source of glutamate protein hydrolysates can be used or freeze dried cultures of (lactic acid) bacteria (probiotics) which contain glutamate as a protecting agent. An example of such a lactic acid culture is *Lactobacillus Reuteri*, obtainable from Biogaia, originating from human milk.

Free glutamic acid is understood to be glutamic acid or a salt thereof that is not bound in protein or peptide and that has either been added or is present in the free amino acid fraction of a protein or hydrolysed protein (hydrolysed proteins usually contain 10 - 20% free amino acids) or is present as a protecting agent in a probiotic lactic acid culture.

The preparations of the invention are preferably combined with suitable prebiotics and probiotics, which have a beneficial effect on the intestinal flora. The prebiotics comprise short or long chain oligosaccharides, in particular galacto-oligosaccharides and fructo-oligosaccharides, branched oligosaccharides, sialyloligosaccharide, nucleotides, protein hydrolysates, sialic acid rich milk products or derivatives thereof, etc.

The nutritional preparation to be prepared according to the invention can be used in the treatment of all conditions where hyperpermeability of the intestinal wall is concerned. Examples of these are food allergy, allergy to internal drugs, sepsis and similar clinical conditions, translocation of pathogenic bacteria through the intestinal wall, endotoxaemia, viral diarrhoea, low intestinal blood flow, IC patients, patients after surgical interventions or with major bums, parenteral nutrition and undernutrition. It can also be used in the case of intestinal maturation of newborn babies, reduction of abnormal crying in children or the treatment of hyperactivity (Attention Deficit Hyperactivity Disorder, ADHD).

### Example 1

The macromolecular permeability of the intestinal epithelium is controlled by the passage through intercellular tight junctions in the paracellular channels. Opening of these tight junctions is controlled by the epithelial cells in response to various intercellular mediators, such as Ca, cyclic AMP, G proteins and protein kinase c. The human intestinal cell line HT-29CL.19A is becoming increasingly more important for studying this paracellular permeability in vitro. See also the abovementioned article in J. *Parenteral Enteral Nutrition*.

For the present examples, confluent monolayers of HT-29CL.19A cells were cultured on permeability filters. After 14 - 17 days the cells were allowed to grow for a further two days without glutamine in the medium. The transepithelial permeability from apical to basolateral was determined for horseradish peroxidase (HRP) with the aid of an enzyme assay. Phorbol 12,13-dibutyrate (PDB, 1 mmol/l) was used to increase the permeability. The effect of glutamine, glutamate and the gamma-glutamyl transferase inhibitor acivicin was investigated. All agents were added to the apical compartment.

It was found that PDB increases the HRP flux 3-fold compared with the control after 150-279 min stimulation (p < 0.001). Glutamine reduces this hyperpermeability appreciably. Glutamate (0.6 mmol/l) had the same effect (p < 0.001). Acivicin prevented the glutamine-mediated reduction in the hyperpermeability induced by PDB. This effect did not occur in the presence of glutamate.

It can be seen from this experiment that glutamate reduces the macromolecular hyperpermeability in HT-29CL.19A cells.

### Example 2

A complete, pulverulent, glutamic acid-containing baby food for premature children was prepared which had the following composition per 100 g powder - dry matter:

| | |
|---|---|
| desalinated whey, solids | 39.2 g |
| vegetable fats | 26.4 g |
| lactose | 17.9 g |
| skimmed milk, solids | 13.4 g |
| glucose syrup | 0.90 g |
| soy lecithin | 0.16 g |
| glutamic acid | 0.5 g |
| L-arginine | 0.05 g |
| taurine | 0.04 g |
| L-Tryptophane | 0.02 g |
| nucleotides | 0.03 g |
| microminerals and vitamins | 1.4 g |
| | |
| casein/whey protein ratio | 40/60 |
| % crude protein | 10.8 |
| % free glutamic acid | 0.5 |

A fluid composition that contains approximately 15% solids can be prepared from such a powder. Approximately 175 ml of the fluid composition is administered per kg body weight per day.

### Example 3

A food was prepared as in example 2, with the exception that instead of 0.7 g lactose 0.7 g galacto-oligosaccharides were incorporated per 100 g powder.

### Example 4

A complete, pulverulent, glutamic acid-containing baby food for children with an allergy was prepared which had the following composition per 100 g powder - dry matter:

| | |
|---|---|
| hydrolysed casein | 13.5 g |
| vegetable fat | 27 g |
| glucose syrup | 58.05 g |
| taurine | 0.04 g |
| L-camitine | 0.01 g |
| microminerals and vitamins | 1.4 g |
| | |
| % crude protein | 12 |
| % free omithin | 0.01 |
| % free glutamic acid | 0.3 |

### Example 5

A food was prepared according to example 4, with the exception that 0.25 g arginin and 0.005 g spermine and spermidine was incorporated instead of 0.255 g glucose syrup.

### Example 6

A pulverent food for young children was prepared to limit excessive crying which contained per 100 gram:

| | |
|---|---|
| lactic protein | 11 g |
| fat | 27 g |
| lactose | 56 g |
| | |
| nucleotides | 0.03 g |
| glutamic acid | 0.45 g |
| minerals, vitamins, probiotic | 3.02 g |
| water | 2.5 g |
| | |
| ratio whey protein : casein : casein hydrolysate 40:30:30 | |
| L. Reuteri (Biogaia) | 1 x 10⁸ |
| | |
| % free glutamic acid | 0.5 |

### Example 7

A pulverent food for older children with multiple functional properties was prepared, containing per 100 grams:

| | |
|---|---|
| lactic protein | 22.1 g |
| fat | 18.4 g |
| lactose | 39 g |
| sucrose | 10 g |
| alfa-ketoglutarate | 0.1 g |
| fructo-oligosaccharide (inulin) | 3 g |
| nucleotides | 0.05 g |
| minerals, vitamins, probiotics | 4.85 g |
| water | 2.5 g |
| | |
| L. Reuteri (Biogaia) | 1 x 10⁸ |
| | |
| casein/whey protein ratio | 75:25 |
| goat's milk protein (comprising human-milk like sialyloligosaccharides) : 20 % of the lactic protein | |
| | |
| % alfa-ketoglutarate | 0.1 |
| % free glutamic acid | 0.05 |
| % free glutamic acid equivalents | 0.15 |

### Example 8

A pulverent food for children having hyperactivity syndrome (ADHD) was prepared containing, compared to example 7, a casein/casein hydrolysate ratio in the lactic protein fraction of 40:60.

The product contains per 100 g

| | |
|---|---|
| L. Reuteri | 1 x 10⁸ |
| % free glutamic acid equivalents | 0.45 |

### Example 9

A problem-solving, fluid, glutamic acid-containing enteral food based on caseinate and glutamine-rich vegetable hydrolysed protein (30% glutamine) was prepared which had the following composition per 100 g:

| | |
|---|---|
| caseinate | 5.2 g |
| glutamine-rich hydrolysed protein | 1.0 g |
| glutamic acid | 0.6 g |
| arginine | 0.2 g |
| fats | 3.4 g |
| carbohydrates | 9.5 g |
| minerals and vitamins | 0.4 g |
| lecithin | 0.1 g |
| water | 79.6 g |
| | |
| % crude protein | 6.3 g |
| % free arginin | 0.2 g |
| % glutamin | 0.3 g |
| % free glutamic acid | 0.6 g |

## Claims

1. Use of glutamate and/or glutamic acid for the preparation of a pharmaceutical composition for the treatment or prevention of suboptimal intestinal wall maturation of new-born human babies by treatment or prevention of hyperpermeability or undesired permeability of the intestinal wall.

2. Use according to claim 1, wherein the pharmaceutical preparation is an enteral food or a food supplement.

3. Use according to claims 1 or 2, wherein the preparation also contains lactic proteins or hydrolysed lactic proteins.

4. Use according to any one of claims 1 to 3, wherein the preparation also contains vegetable proteins or hydrolysed products thereof.

5. Use according to any one of claims 1 to 4, wherein hydrolysed protein is used as the source of glutamate.

6. Use according to any one of the preceding claims, wherein the preparation also contains glutamine or an equivalent thereof.

7. Use according to claim 6, wherein the weight ratio of glutamic acid : glutamine in the free amino acid fraction is greater than 1 : 1, in particular greater than 5 : 1 and preferentially greater than 25 : 1.

8. Use according to any one of the preceding claims, wherein the preparation further contains one or more polyamines, in particular spermine, spermidine or putrescine and/or one or more polyamine precursor, in particular ornithin and arginin.

9. Use according to any one of the preceding claims, wherein the nutritional preparation contains 0. 05-10 g, preferably 0. 2-4 g free glutamate per 100 g preparation (dry weight).

10. Use according to any one of the preceding claims, wherein the preparation further contains one or more prebiotics, preferably selected from the group consisting of protein hydrolysates, nucleotides, galacto-oligosaccharides, fructo-oligosaccharides, branched oligosaccharides and sialyloligosaccharides or equivalents thereof.

11. Use according to any one of the preceding claims, wherein the preparation contains freeze dried probiotics, in particular freeze dried *Lactobacillus reuteri* as the source of glutamate.

## Patentansprüche

1. Verwendung von Glutamat und/oder Glutaminsäure zur Herstellung einer pharmazeutischen Zusammensetzung für die Behandlung oder Verhinderung von suboptimaler Darmwandreifung von neugeborenen menschlichen Säuglingen durch Behandlung oder Verhinderung von Hyperpermeabilität oder unerwünschter Permeabilität der Darmwand.

2. Verwendung gemäß Anspruch 1, wobei die pharmazeutische Zubereitung eine enterale Nahrung oder eine Nahrungsergänzung ist.

3. Verwendung gemäß Ansprüchen 1 oder 2, wobei die Zubereitung auch Milchproteine oder hydrolysierte Milchproteine enthält.

4. Verwendung gemäß einem der Ansprüche 1 bis 3, wobei die Zubereitung auch pflanzliche Proteine oder hydrolysierte Produkte davon enthält.

5. Verwendung gemäß einem der Ansprüche 1 bis 4, wobei hydrolysiertes Protein als Glutamatquelle verwendet wird.

6. Verwendung gemäß einem der vorhergehenden Ansprüche, wobei die Zubereitung auch Glutamin oder ein Äquivalent davon enthält.

7. Verwendung gemäß Anspruch 6, wobei das Gewichtsverhältnis von Glutaminsäure : Glutamin in der Fraktion der freien Aminosäuren größer als 1 : 1, insbesondere größer als 5 : 1 und vorzugsweise größer als 25 : 1 ist.

8. Verwendung gemäß einem der vorhergehenden Ansprüche, wobei die Zubereitung ferner ein oder mehrere Polyamin(e), insbesondere Spermin, Spermidin oder Putrescin, und/oder eine oder mehrere Polyaminvorstufe(n), insbesondere Ornithin und Arginin, enthält.

9. Verwendung gemäß einem der vorhergehenden Ansprüche, wobei die Nahrungszubereitung 0,05-10 g, vorzugsweise 0,2-4 g freies Glutamat pro 100 g der Zubereitung (Trockengewicht) enthält.

10. Verwendung gemäß einem der vorhergehenden Ansprüche, wobei die Zubereitung ferner eine oder mehrere prebiotische Substanz(en), vorzugsweise ausgewählt aus der Gruppe bestehend aus Proteinhydrolysaten, Nukleotiden, Galacto-Oligosacchariden, Fructo-Oligosacchariden, verzweigten Oligosacchariden und Sialyloligosacchariden oder Äquivalenten davon, enthält.

11. Verwendung gemäß einem der vorhergehenden Ansprüche, wobei die Zubereitung gefriergetrocknete Probiotika, insbesondere gefriergetrockneten *Lactobacillus reuteri,* als Glutamatquelle enthält.

## Revendications

1. Utilisation de glutamate et/ou d'acide glutamique pour la préparation d'une composition pharmaceutique en vue du traitement ou de la prévention de la maturation de la paroi intestinale suboptimale chez les bébés humains nouveaux nés par le traitement ou la prévention de l'hyperperméabilité ou de la perméabilité non désirée de la paroi intestinale.

2. Utilisation selon la revendication 1, dans laquelle la préparation pharmaceutique est un aliment entéral ou un produit de supplémentation alimentaire.

3. Utilisation selon l'une quelconque des revendications 1 à 2, dans laquelle la préparation contient également des protéines lactiques ou des protéines lactiques hydrolysées.

4. Utilisation selon l'une quelconque des revendications 1 à 3, dans laquelle la préparation contient également des protéines végétales ou ces produits hydrolysés.

5. Utilisation selon l'une quelconque des revendications 1 à 4, dans laquelle une protéine hydrolysée est employée comme source de glutamate.

6. Utilisation selon l'une quelconque des revendications précédentes, dans laquelle la préparation contient également de la glutamine ou un équivalent de celle-ci.

7. Utilisation selon la revendication 6, dans laquelle le rapport en poids acide glutamique/glutamine dans la fraction d'acide aminé libre est supérieure à 1:1, en particulier supérieure à 5:1 et préférentiellement supérieure à 25:1.

8. Utilisation selon l'une quelconque des revendications précédentes, dans laquelle la préparation contient en outre un ou plusieurs polyamines, en particulier de la spermine, de la spermidine ou de la putrescine et/ou un ou plusieurs précurseur(s) de polyamine, en particulier de l'ornithine et de l'arginine.

9. Utilisation selon l'une quelconque des revendications précédentes, dans laquelle la préparation contient de 0,05 à 10 g, de préférence de 0,2 à 4 g de glutamate libre pour 100 g de préparation (poids sec).

10. Utilisation selon l'une quelconque des revendications précédentes, dans laquelle la préparation contient en outre un ou plusieurs prébiotique(s), de préférence choisi(s) parmi le groupe constitué des hydrolysats de protéine, des nucléotides, des galacto-oligosaccharides, des fructo-oligosaccharides, des oligosaccharides ramifiés et des sialyloligosaccharides ou des équivalents de ceux-ci.

11. Utilisation selon l'une quelconque des revendications précédentes, dans laquelle la préparation contient des probiotiques lyophilisés, en particulier du *Lactobacillus reuteri* lyophilisé comme source de glutamate.
